(19) Europäisches Patentamt
European Patent Office
Office européen des brevets

(11) Publication number: **0 158 513**
**A2**

(12) # EUROPEAN PATENT APPLICATION

(21) Application number: 85302376.0

(22) Date of filing: 12.04.85

(51) Int. Cl.⁴: C 07 D 473/32
C 07 D 473/40, A 61 K 31/52

(30) Priority: 13.04.84 GB 8409736

(43) Date of publication of application: 16.10.85 Bulletin 85/42

(84) Designated Contracting States: AT BE CH DE FR GB IT LI LU NL SE

(71) Applicant: THE WELLCOME FOUNDATION LIMITED
183-193 Euston Road
London NW1 2BP(GB)

(72) Inventor: Beauchamp, Lilia Marie
3007 Wycliff Road
Raleigh North Carolina 27607(US)

(72) Inventor: Krenitsky, Thomas Anthony
106 Laurel Hill Road
Chapel Hill North Carolina 27514(US)

(74) Representative: Garrett, Michael et al,
*The Wellcome Foundation Limited Group Patents and Agreements Langley Court*
Beckenham Kent BR3 3BS(GB)

(54) **Antiviral compounds.**

(57) Compounds of the general fomula

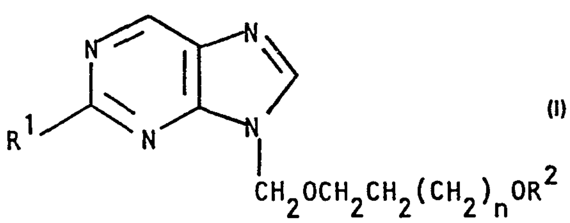

(I)

(wherein $R^1$ represents a chlorine atom or an amino group, n is 0 or 1, $R^2$ represents a pentanoyl, hexanoyl, 2-carboxypropionyl or benzyl group when n is 0 or $R^2$ represents a hydrogen atom or $C_{1-6}$ alkanoyl group when n is 1) have been found to have valuable antiviral properties particularly against viruses of the herpes family such as herpes simplex.

EP 0 158 513 A2

Croydon Printing Company Ltd

## Antiviral Compounds

The present invention relates to antiviral purine derivatives containing an acyclic chain in the 9-position.

U.K. Patent Specification No. 1523865 describes a broad class of purine derivatives containing an acyclic side chain in the 9-position. These purine derivatives have been found to have antiviral activity against various classes of DNA viruses particularly against herpes viruses such as herpes simplex.

Among these derivatives, 9-(2-hydroxyethoxymethyl)guanine (otherwise known as acyclovir) has been found to have particularly good activity against herpes viruses such as herpes simplex. However, while acyclovir has been found to be especially effective upon topical or parenteral administration, it is only moderately well absorbed upon oral administration with corresponding levels of drug in the plasma. It will be appreciated that when one is treating an internal disorder by oral administration of a drug, it is clearly desirable that the drug should be well absorbed from the gastro-intestinal tract with resulting high plasma levels.

In UK Patent Specification 2130204 we describe certain purine derivatives which are characterised by the presence of a hydrogen atom in the 6- position of the purine nucleus, and which can be readily converted in vivo by the action of enzymes of the molybdo-flavo-protein type (especially xanthine oxidase/dehydrogenase or aldehyde oxidase) into the corresponding 6-hydroxy purine derivatives having antiviral activity. Furthermore, from experiments in rats, we have found that oral administration of such 6-hydrogen derivatives results in efficient absorption from the gastro-intestinal tract and high plasma levels of the corresponding 6-hydroxy compound, formed by enzymatic conversion of the 6-hydrogen compound. Also, for example, 6-deoxyacyclovir, i.e. 2-amino-9-(2-hydroxyethoxymethyl)purine, the 6-hydrogen analogue of acyclovir, is also considerably more soluble in water than acyclovir, the former compound having a solubility of 50mg/ml and the latter having a solubility of 1.23mg/ml at 25$^{o}$C. This improved water-solubility enables 6-deoxyacyclovir to be used in a greater variety of aqueous pharmaceutical formulations which require some solubilisation of the drug.

The above-mentioned class of 6-hydrogen purine derivatives described in the said UK Patent Specification may be represented by the general formula

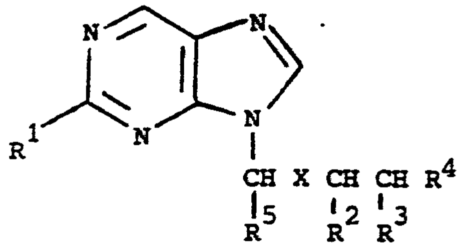

(IA)

wherein X is sulphur or oxygen or a chemical bond; $R^1$ is halogen, amino or azido; $R^2$ is hydrogen, hydroxy, alkyl or hydroxyalkyl; $R^3$ is hydrogen or alkyl; $R^4$ is hydroxy, hydroxyalkyl, benzyloxy, phosphate or carboxypropionyloxy; $R^5$ is hydrogen, alkyl or hydroxyalkyl; and esters of those compounds wherein $R^2$ is hydroxy or hydroxyalkyl; $R^4$ is hydroxy, hydroxyalkyl or carboxypropionyloxy or $R^5$ is hydroxyalkyl; and physiologically acceptable salts of such compounds of formula (I) and their esters.

A particularly preferred compound of formula (IA) is 2-amino-9-(2-hydroxy ethoxymethyl)-9H-purine.

According to the present invention we provide compounds of the general formula

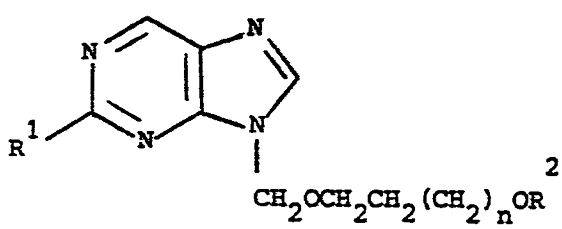

(I)

wherein $R^1$ represents a chlorine atom or an amino group, n is 0 or 1, $R^2$ represents a pentanoyl (e.g. pivaloyl), hexanoyl, 2-carboxypropionyl or benzyl group when n is 0, or $R^2$ represents a hydrogen atom or a $C_{1-6}$ alkanoyl e.g. acetyl group when n is 1.

Such compounds of formula (I) include the following specific compounds:-

2-[(2-amino-9H-purin-9-yl)methoxy]ethyl pivalate
2-[(2-amino-9H-purin-9-yl)methoxy]ethyl n-hexanoate
2-[(2-amino-9H-purin-9-yl)methoxy]ethyl hydrogen succinate
3-[(2-chloro-9H-purin-9-yl)methoxy]propyl acetate

2-amino-9-(3-hydroxypropoxymethyl)-9H-purine
2-amino-9-(2-benzyloxyethoxymethyl)-9H-purine

In addition to the conversion of the 6-hydrogen atom into a 6-hydroxy group by enzymatic action in vivo, as referred to above, the ester compounds of formula (I) are also converted into the corresponding free alcohols. Thus, when ester compounds of formula (I) are administered, especially orally, higher blood levels of the corresponding non-esterified 6-hydroxy compounds are obtained and maintained for substantially longer periods of time than when the latter compounds are administered directly.

The discovery that the 6-hydrogen purines of formula (IA) referred to above can be readily converted into their corresponding 6-hydroxy analogues was surprising since in previous studies with xanthine oxidase from bovine milk (H.Lettre et al (1967) Biochem.Pharmacol., 16, 1747-1755; T.A. Krenitsky et al (1972) Arch. Biophys., 150, 585-599), it was shown that 9- substitution obliterates or greatly diminishes the rate at which a variety of purines are oxidised. In view of these observations, it was surprising to find that this enzyme oxidised for example 6-deoxyacyclovir, a 9-substituted derivative of 2-aminopurine, at a faster rate than the 9-unsubstituted purine, as we have established from enzyme studies.

The high level of absorption of the compounds of formula (I) from the gastro-intestinal tract renders the compounds especially useful when oral administration of the compounds is desired, e.g. in the treatment of diseases caused by various DNA viruses, such as herpes infections for example herpes simplex, varicella or zoster, cytomegalovirus as well as diseases caused by hepatitis B or Epstein-Barr virus. The compounds of formula (I) can also be used for the treatment or prophylaxis of papilloma or wart virus infections. In addition to their use in human medical therapy the compounds of formula (I) can be administered to other animals for the treatment or prophylaxis of viral diseases, e.g. in other mammals, for example, the treatment of equine rhinopneumonitis. By administration of a compound of formula (I) or physiologically acceptable salt or ester thereof, especially by the oral route, it is possible to achieve an advantageous effect against such disorders.

According to a further feature of the present invention we provide compounds of formula (I) for use in the treatment or prophylaxis of a viral disease in an animal, e.g. a mammal such as man.

The present invention also provides a method for the treatment or prophylaxis of a viral disease in an animal, e.g. a mammal such as man which comprises administering to the animal an effective antiviral amount of a compound of formula (I).

The compounds of formula (I) (hereafter collectively referred to as the active ingredients) may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general however, for each of these utilities and indications, a suitable, effective dose will be in the range 0.1 to 250mg per kilogram bodyweight of recipient per day, preferably in the range 1 to 100mg per kilogram bodyweight per day and most preferably in the range 5 to 20mg per kilogram bodyweight per day; an optimum dose is about 10mg per kilogram bodyweight per day. The desired dose is preferably presented as two, three, four or more sub-doses administered at appropriate intervals throughout the day. These sub-doses may be administered in unit dosage forms, for example, containing 10 to 1000mg, preferably 20 to 500mg and most preferably 100 to 400mg of active ingredient per unit dosage form.

While it is possible for the active ingredients to be administered alone it is preferable to present them as pharmaceutical formulations. The formulations, both for veterinary and for human use, of the present invention comprise at least one active ingredient, as above defined, together with one or more pharmaceutical carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage

form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described. Preferred unit dosage formulations are those containing a daily dose or unit daily sub-dose, as herein above recited, or an appropriate fraction therof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

For oral administration the compositions can be in the form of a tablet, granule, drench, paste, cachet, capsule or feed supplement. Granules may be made by the well known techniques of wet granulation, precompression or slugging. They can be administered to animals in an inert liquid vehicle so as to form a drench, or in a suspension with water or oil base. Preferably further accessory ingredients such as a dispensing agent are included. These formulations preferably contain from 15 to 85% of the active ingredient.

A paste may be formulated by suspending the active ingredient in a liquid diluent. A stiffening or thickening agent may be included together with a wetting agent or a humectant if the liquid diluent is water. If an emulsion paste is needed then one or more surface active agents should desirable in included. From 25 to 80% by weight of these paste formulations may comprise the active ingredient.

In feed supplements the active ingredient is generally present in large amounts relative to the accessory ingredients, and the supplements may be added directly or after intermediate blending or dilution. Examples of accessory ingredients for such formulations include solid, orally ingestible carriers such as corn meal, soya flour, wheat shorts, soya grits, edible vegetable materials and fermentation residues. The active ingredient is usually incorporated in one or more of the accessory ingredients and intimately and uniformly dispersed by grinding, tumbling or stirring with conventional apparatus. Formulations containing 1 to 90% by weight of the active ingredient are suitable for adding to feeds.

For the treatment of herpes infections in horses, an oral or parenteral dose of from 0.1 to 250mg per kg body weight per day, preferably from 2 to 100mg per kg per day may be required. The dose may be split up into discrete units administered at regular intervals during the day, and repeated daily for up to 14 days or until the infection is cleared. For viral infections in other animals the dose may vary depending on the size and metabolism of the animal. The compositions may be administered in unit dosage form, such as a tablet a few times daily in the amount of 10 to 1000mg per unit dose.

The compounds of formula (I) may be prepared in conventional manner by analogous processes for preparing compounds of similar structure, such as those methods described in U.K. Patent Specifications Nos. 1523865 and 2130204.

According to a further feature of the present invention we provide a process for preparing compounds of formula (I) (wherein $R^2$ is other than a hydrogen atom or a benzyloxy group) which comprises reacting 2-amino-9-(2-hydroxyethoxy methyl)-9H-purine with an appropriate esterifying agent., e.g. using the corresponding acid anhydride.

Alternative processes for preparing compounds of formula (I) include:-

a) deblocking a corresponding compound substituted in the 2-position of the purine nucleus by a blocked amino group;

b) converting a compound of formula

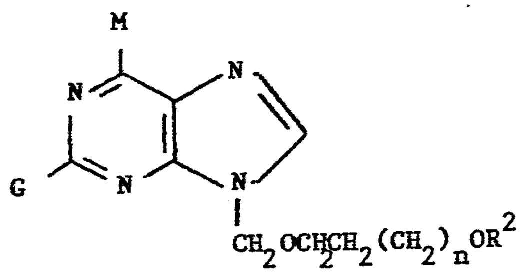

(II)

(wherein $R^2$ and n are as defined above, M represents a hydrogen atom or a group or atom convertible into a hydrogen atom and G represents a group or atom convertible into a chlorine atom or an azido or amino group or (when M is other than a hydrogen atom) G may alternatively represent an amino group) into a compound of formula (I);

c) reacting a compound of formula

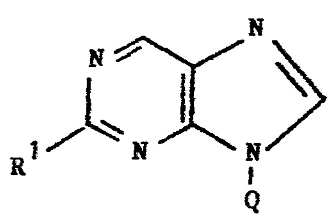

(III)

(wherein Q represents a leaving atom or group) with a compound of formula

(IV)

$$ACH_2OCH_2CH_2(CH_2)_nOR^2$$

(wherein n and $R^2$ are as defined above and A represents a leaving group or atom)

In method a) the blocking group may be selected, as appropriate, for example from acyl groups such as $C_{1-4}$alkanoyl groups e.g. acetyl or pivaloyl, or aroyl groups, e.g. benzoyl; arylmethyl groups e.g. benzyl; or tri-$C_{1-4}$alkylsilyl e.g. trimethylsilyl. Arylmethyl blocking groups, may be removed for example by hydrogenolysis, e.g. by hydrogenation in the presence of Raney nickel or palladium catalyst or by the use of sodium in liquid ammonia. Acyl blocking groups may be removed for example by hydrolysis using for example an amine such as methylamine or triethylamine, advantageously in an aqueous medium. Trialkylsilyl blocking groups may be removed for example by solvolysis e.g. with alcoholic or aqueous ammonia, or by alcoholysis.

Conversion of a compound of formula (II) into a compound of formula (I), by method b), can be achieved by various conventional means. For example G may represent an azide group which can be reduced to an amino group by catalytic hydrogenation using a suitable catalyst such as palladium. Alternatively, G may represent a halogen atom or an alkylthio or alkylsulphonyl group which can be converted to an amino group by aminolysis using for example ammonia. M may represent a halogen e.g. chlorine, atom or a mercapto or thio (S <) group which can be converted into a hydrogen atom in conventional manner. In the case where M represents a thio group, this conversion may be effected using a compound of formula (II) in which any amino or hydroxyl groups are optionally blocked by acyl groups, the conversion being effected using a Raney nickel catalyst, e.g in a basic medium which additionally removes the amino and/or hydroxy blocking groups in accordance with process a).

These processes together with other conventional processes are described in Fused Pyrimidines, Part II, Purines Ed. by D. J. Brown (1971), Wiley-Interscience.

In process (c), the group Q in formula (III) may for example represent a hydrogen atom; an acyl group, e.g. a $C_{1-4}$alkanoyl group such as an acetyl group or an aroyl group such as a benzoyl group; or a tri-$C_{1-4}$alkylsilyl group such as a trimethylsilyl group. The group A in formula (IV) may for example represent a halogen atom (e.g. chlorine) or an acyloxy group wherein the acyl moiety may be for example a $C_{1-4}$alkanoyl group such as acetyl, or an aroyl group such as benzoyl. The reaction may be conveniently effected in a strong polar solvent such as dimethylformamide or hexamethylphosphoramide, advantageously in the presence of a base such as triethylamine or potassium carbonate. Alternatively, a thermal condensation may be effected by heating the compounds of formulae (III) and (IV) in the presence of a catalytic amount of a strong acid, e.g. sulphuric acid.

The starting materials employed in the processes described above may be prepared in conventional manner, e.g. in accordance with the processes described in the above-mentioned UK Patent Specifications 1523865 and 2130204.

The following Examples illustrate the present invention:-

## Example 1

2-[(2-Amino-9H-purin-9-yl)methoxy]ethyl pivalate

A mixture of 1.92 g (9.15 mM) of 2-[(2-amino-9H-purin-9-yl)methoxy]ethanol and 29 ml of dry dimethylformamide was heated on a steam bath until solution occurred, then combined, on cooling to room temperature, with 2.2 ml dry pyridine, 0.34g of 4-dimethylaminopyridine and 5.6 ml of pivalic anhydride. The solution was stirred at room temperature for 18 hours, flash evaporated and purified by column chromatography on silica gel. The column was eluted successively with 20%, then 50% acetone in ether and finally neat acetone, the latter two eluates producing on evaporation, 2.25 g of the desired product which was recrystallized from benzene-hexane to yield 2-[(2-amino-9H-purine-9-yl)methoxy]ethyl pivalate, 1.72 g (64%), m.p. 113.5-115$^{o}$C. The $^{1}$H NMR spectrum was consistent with the desired structure.

Example 2

2-[(2-Amino-9H-purin-9-yl)methoxy]ethyl n-hexanoate

A mixture of 3.0 g (14 mM) of 2-[(2-amino-9H-purin-9-yl)methoxy]ethanol and 40 ml of dry dimethylformamide was combined with 6.5 ml (28 mM) of n-hexanoic anhydride, 1.8 ml (22.26 mM) of pyridine, and 1.5 g (12 mmole) of 4-dimethylaminopyridine and then stirred under nitrogen at room temperature for nearly seven hours. The solution was washed with $H_2O$ and the product extracted from the aqueous solution with ethyl acetate. The ethyl acetate layer was dried over $Na_2SO_4$, then filtered and evaporated to give a thin liquid. This was dissolved in ethyl acetate, and pentane was added until cloudiness appeared. Analytically pure 2-[2-amino-9H-purin-9-yl)methoxy]-ethyl n-hexanoate was obtained as a white solid weighing 3.6 g (11.71 mM), m.p. 105-107°C (84% yield). The 'H NMR spectrum was consistent with the desired structure.

Example 3

3-[(2-Chloro-9H-purin-9-yl)methoxy]propyl acetate

A mixture of 0.68 g (8.3 mM) of sodium acetate in 2ml $H_2O$, 50 ml absolute ethanol and 0.26 g of 5% Pd-C was shaken under 50 psi $H_2$ and the uptake of $H_2$ measured. To this preconditioned mixture was added 1.0 g (3.0 mM) of 9-(3-acetoxypropoxymethyl)-2,6-dichloropurine in 30 ml of absolute ethanol. The combined mixture was shaken at room temperature under the initial pressure of 20 psi $H_2$ for 84 minutes during which time the uptake of $H_2$ was 6.0 pounds.

The reaction mixture was removed from the apparatus, filtered through a pad of Celite and the filtrate evaporated in vacuo to yield a white solid. The solid was extracted with boiling acetone and the acetone extracts evaporated and purified on a silica gel column, eluting initially with dichloromethane, then with progressively increasing mixtures of ether in dichloromethane, 100% ether and finally acetone. The $CH_2Cl_2$ and the 10-20% $Et_2O$-$CH_2Cl_2$ eluates yielded the starting material (0.2 g) and the 30-100% $Et_2O$-$CH_2Cl_2$ mixtures gave on evaporation a residue which was recrystallized from benzene-hexane to produce the title compound, 245 mg as analytically pure material, m.p. 79-81°C, 36% yield based on amount of starting material reduced.

## Example 4

2-[(2-Amino-9H-purin-9-yl)methoxy]ethyl hydrogen succinate

A mixture of 0.95 g (4.5 mM) of 2-amino-9-(2-hydroxyethoxymethyl)-purine, 16 ml of dry dimethylformamide, 55 mg (0.45 mM) of 4-dimethylaminopyridine and 0.9 g (9.0 mM) of succinic anhydride was stirred at room temperature for four days. The clear solution was evaporated in vacuo and the resulting residual oil triturated with ethyl acetate and recrystallized from hot methanol, absorbed on silica gel and added, on evaporation, to a column prepared for flash chromatography. The column was eluted with 20% methanol in dichloromethane, and the evaporated eluates recrystalized from methanol to yield 320 mg (23%) of the title compound as analytically pure material (half hydrate) mp 120-123$^{o}$C. The $^{1}$H NMR and UV spectra were consistent with the assigned structure.

## Example 5

a) 3-[(2-Azido-9H-purin-9-yl)methoxy]propyl acetate

To a refluxing mixture of 100 mg (0.35 mM) of 3-[(2-chloro-9H-purin-9-yl) methoxy]propyl acetate in 10 ml of 1:1 v/v EtOH-$H_2O$ is added in one portion, 23 mg (0.36 mM) of sodium azide. The mixture is refluxed for four hours, evaporated in vacuo and triturated with water. The residue is recrystallized from ethanol to yield the title compound.

b) 2-Amino-9-[(3-hydroxypropoxy)methyl]-9H-purine

A mixture of 0.8g (2.75mM) of 3-[(2-azido-9H-purin-9-yl)methoxy]propyl acetate and 0.4g of 5% palladium on charcoal in 300ml of methanol is shaken on a Parr hydrogenator under an initial pressure of 50 p.s.i. for 18 hours at room temperature. The mixture is filtered through a pad of Celite and the pad washed with additional methanol. The combined filtrate and washings are heated on a steam bath with 40ml of 40% aqueous methylamine for thirty minutes then evaporated in vacuo. Recrystallisation of the residue from ethanol yields the title compound. The $^{1}$H NMR and ultraviolet spectra are consistent with the proposed structure.

Example 6

a) 2-Amino-9-[(2-benzyloxyethoxy)methyl]-6-chloro-9H-purine

A mixture of 5.0g (16.6 mM) of 2-amino-6-chloropurine, 4.07g of potassium carbonate, 4.66g (23.2 mM) of 2-benzyloxyethoxymethyl chloride and 100ml of dry dimethylformamide was stirred at room temperature for seven days. The reactant mixture was filtered, the solid washed with ether and the combined filtrate and washings evaporated in vacuo. The residual oil was purified by column chromatography on silica gel, eluting the desired 9-isomer with 10% methanol in dichloromethane. The fractions were monitored by TLC, cooled and evaporated to yield a yellow oil, which was further purified by column chromatography, eluting with ethyl acetate to yield 0.437 g (5.6%) of the title compound. The NMR spectrum is consistent with the structure.

b) 2-Amino-1,9-dihydro-9-[(2-benzyloxyethoxy)methyl]-6H-purine-6-thione

A mixture of 1.22g (3.66 mM) of 2-amino-9-[(2-benzyloxyethoxy)methyl]-6-chloro-9H purine, 40ml of isopropanol and 0.28g of dried (1 hour at $100^{0}C$) thiourea was refluxed on a steam bath for 1.5 hours. The reaction mixture was filtered, triturated with isopropanol and recrystallised once from ethanol and once from aqueous ethanol to yield the title compound, m.p. 217-$218^{0}C$., 0.144g (34%). An additional crop was obtained by concentration of the mother liquor.

c) 2-amino-9-[(2-benzyloxyethoxy)methyl]-9H-purine

A mixture of 0.79g (2.38 mM) of 2-amino-1,9-dihydro-9-[(2-benzyloxyethoxy)-methyl]-6H-purine-6-thione, 4g of Raney Nickel, 100ml of water, 3ml of ammonium hydroxide (conc.) and 20ml of ethanol is refluxed with stirring for 18 hours. The mixture is filtered and the nickel extracted with hot ethanol. The combined filtrate and ethanolic extracts are evaporated in vacuo and the residue recrystallised from isopropanol to give title compound. The elemental analysis and $^{1}H$ NMR spectrum are consistent with the desired structure.

The following Examples illustrate pharmaceutical formulations according to the invention.

**Example 7** **Tablet**

| | |
|---|---|
| Active compound | 200mg |
| Lactose | 235mg |
| Starch | 50mg |
| Polyvinylpyrrolidone | 50mg |
| Magnesium stearate | 5mg |
| | 500mg |

Mix the active compound with the lactose and starch and wet granulate with a solution of the polyvinlpyrrolidone. Dry, sift, blend the granules with magnesium stearate and compress.

**Example 8** **Capsule**

| | |
|---|---|
| Active compound | 200mg |
| Lactose | 184mg |
| Sodium starch glycollate | 8mg |
| Polyvinylpyrrolidone | 6mg |
| Magnesium stearate | 2mg |

Mix the active compound with the lactose and sodium starch glycollate and wet granulate with a solution of the polyvinylpyrrolidone. Dry, sift, blend the granules with the magnesium stearate and fill ino hard gelatin capsules.

**Example 9** **Intravenous Injections**

| | | | |
|---|---|---|---|
| A) | Active compound | | 200mg |
| | Sodium hydroxide solution | q.s. to pH 7.0 to 7.5 | |
| | Water for injections | to | 5.0 ml |

Dissolve the active compound in part of the water for injections. Adjust the pH with the sodium hydroxide solution and make up to volume with additional water for injections. Under aseptic conditions, sterilise the solution by filtration, fill into sterile ampoules and seal the ampoules.

| | | | |
|---|---|---|---|
| B) | Active compound | | 100mg |
| | Sodium hydroxide solution | q.s. to pH 7.0 to 7.5 | |
| | Mannitol | | 125mg |
| | Water for injections | to | 2.5ml |

Dissolve the active compound and mannitol in part of the water for injections. Adjust the pH with the sodium hydroxide solution and make up to volume with additional water for injections. Under aseptic conditions, sterilise the solution by filtration, fill into sterile vials and remove the water by freeze-drying. Seal the vials under an atmosphere of nitrogen and close with a sterile stopper and aluminium collar.

Antiviral Activity

a) 50 CDI mice were infected with the ICI strain of herpes simplex virus type 1 (innoculation titre: $5 \times 10^5$ pfu.ml) administered in a dose of 0.025ml intracerebrally. The mice were divided into groups of 10, each group receiving respectively 3.125 mg/kg, 6.25 mg/kg, 12.5 mg/kg, 25 mg/kg or 50 mg/kg of the compound of Example 1, administered orally in 0.1 ml volumes, treatment commencing 2 hours after infection and continuing twice daily for 4.5 days. 10 untreated (infected) mice acted as controls. Observations were made over 14 days and survival times were recorded and expressed as mean reciprocal survival times (MRST). The results are shown below.

| Treatment Group | MRST |
|---|---|
| Virus controls | 0.255 |
| 3.125 mg/kg | 0.306 |
| 6.25 | 0.288 |
| 12.5 | 0.236 |
| 25 | 0.213 |
| 50 | 0.216 |

b) Using the same procedure described in a), except that 12 mice are used in the control group, the compound of Example 2 was testing for antiviral activity. The results are shown below.

| Treatment Group | MRST |
| --- | --- |
| Virus Controls | 0.333 |
| 3.125 mg/kg | 0.268 |
| 6.25 | 0.249 |
| 12.5 | 0.233 |
| 25 | 0.243 |
| 50 | 0.219 |

c) The compounds of Examples 1 and 2 were also tested in rats to determine their absorption from the gastro-intestinal tract and conversion into acyclovir (ACV). The compounds were administered in water to Long-Evans rats, the compound of Example 1 being in solution and the compound of Example 2 being in suspension. Further details of the testing and also the results are given in the following table.

| Example | Dose (mg/kg) | Equiv.Dose to ACV (mg/kg) | Rat | Collection Time Post-Dose (hr) | Recovery of ACV % Dose | |
| --- | --- | --- | --- | --- | --- | --- |
| | | | | | RIA | HPLC |
| 1 | 32.6 | 25.0 | 1 | 0-24 | 58.9 | 61.8 |
| | | | | 24-48 | 1.9 | |
| | | | | Total | 60.8 | |
| | | | 2 | 0-24 | 54.3 | 55.3 |
| | | | | 24-48 | 1.1 | |
| | | | | Total | 55.4 | |
| 2 | 34.1 | 25.1 | 1 | 0-24 | 35.3 | |
| | | | | 24-48 | 0.8 | |
| | | | | Total | 36.1 | |
| | | | 2 | 0-24 | 30.0 | |
| | | | | 24-48 | 0.7 | |
| | | | | Total | 30.7 | |

CLAIMS

1. Compounds of the general formula

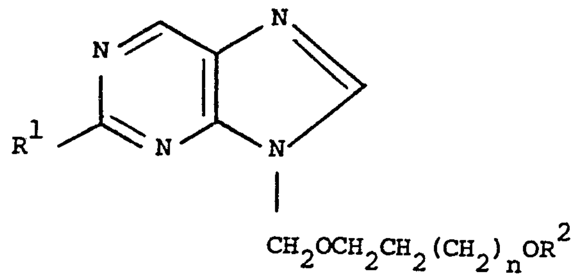

(I)

wherein $R^1$ represents a chlorine atom or an amino group, n is 0 or 1, $R^2$ represents a pentanoyl, hexanoyl, 2-carboxypropionyl or benzyl group when n is 0, or $R^2$ represents a hydrogen atom or a $C_{1-6}$ alkanoyl group when n is 1.

2. Compounds as claimed in claim 1 in which n is 0 and $R^2$ represents a pivaloyl or n-hexanoyl group.

3. 2-[(2-Amino-9H-purin-9-yl)methoxy]ethyl pivalate

4. 2-[(2-Amino-9H-purin-9-yl)methoxy]ethyl n-hexanoate

5. Compounds of general formula (I) (as defined in claim 1) for use in the treatment or prophylaxis of a viral disease in an animal.

6. Compounds as claimed in claim 5 for use in the treatment or prophylaxis of a herpes viral disease.

7. Pharmaceutical formulations comprising at least one compound of formula (I) (as defined in claim 1) together with at least one pharmaceutical carrier or excipient.

8. Pharmaceutical formulations as claimed in claim 7 adapted for oral administration.

9. Pharmaceutical formulations as claimed in claim 7 or claim 8 containing a compound as claimed in claim 3 or claim 4.

MG/MHD/4th March 1985

0158513

10. Use of a compound of general formula (I) (as defined in claim 1) for the preparation of a pharmaceutical formulation for the treatment or prophylaxis of a viral disease in an animal.

11. A process for preparing compound of formula (I) (as defined in claim 1) which comprises

(a) reacting 2-amino-9-(2-hydroxyethoxymethyl)-9H-purine with an appropriate esterifying agent to form a corresponding ester of formula (I);

(b) deblocking a corresponding compound substituted in the 2-position of the purine nucleus by a blocked amino group.

(c) converting a compound of formula

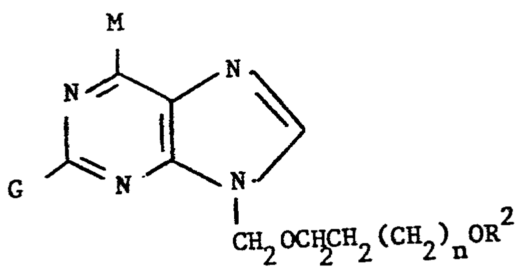

(II)

(wherein R, Z and n are as defined above, M represents a hydrogen atom or a group convertible into a hydrogen atom and G represents a group or atom convertible into a chlorine atom or an amino group or (when M is other than a hdrogen atom) G may alternatively represent an amino group) into a compound of formula (I);

d) reacting a compound of formula

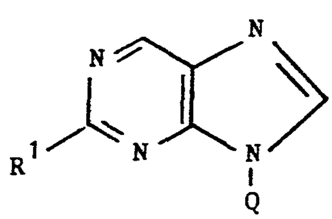

(III)

(wherein Q represents a leaving atom or group) with a compound of formula

(IV)

$$ACH_2OCH_2CH_2(CH_2)_nOR^2$$

(wherein n and $R^2$ are as defined above and A represents a leaving group or atom).

B416CP
0158513

CLAIMS (for Austria)

1. A process for the preparation of compounds of the general formula

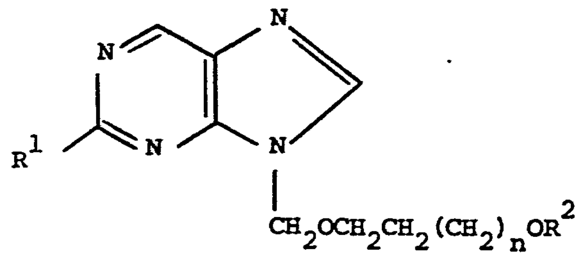

(I)

(wherein $R^1$ represents a chlorine atom or an amino group, n is 0 or 1, $R^2$ represents a pentanoyl, hexanoyl, 2-carboxypropionyl or benzyl group when n is 0, or $R^2$ represents a hydrogen atom or a $C_{1-6}$ alkanoyl group when n is 1) which comprises

(a) reacting 2-amino-9-(2-hydroxyethoxymethyl)-9H-purine with an appropriate esterifying agent to form a corresponding ester of formula (I);

(b) deblocking a corresponding compound substituted in the 2-position of the purine nucleus by a blocked amino group.

(c) converting a compound of formula

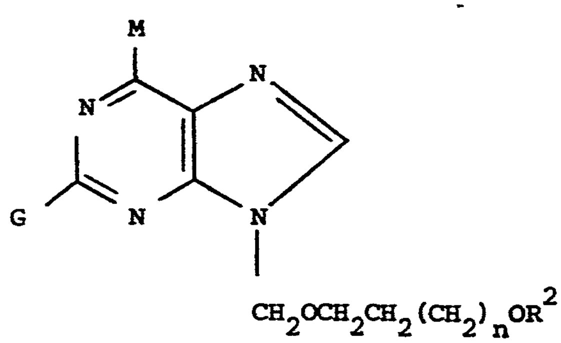

(II)

(wherein R, Z and n are as defined above, M represents a hydrogen atom or a group convertible into a hydrogen atom and G represents a group or atom convertible into a chlorine atom or an amino group or (when M is other than a hydrogen atom) G may alternatively represent an amino group) into a compound of formula (I);

d) reacting a compound of formula

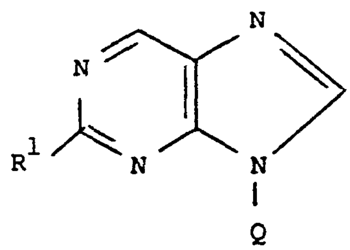

(III)

(wherein Q represents a leaving group or atom) with a compound of formula

$$ACH_2OCH_2CH_2(CH_2)_nOR^2 \quad (IV)$$

(wherein n and $R^2$ are as defined above and A represents a leaving group or atom).

2. A process as claimed in claim 1 (a) in which esterification is effected with an appropriate acid anhydride.

3. A process claimed in claim 1 (a) in which 2-amino-9-(2-hydroxyethoxymethyl)-9H-purine is reacted with pivalic anhydride to form 2-[(2-amino-9H-purin-9-yl)methoxy]ethyl pivalate.

4. A process claimed in claim 1 (a) in which 2-amino-9-(2-hydroxyethoxymethyl)-9H-purine is reacted with n-hexanoic anhydride to form 2-[(2-amino-9H-purin-9-yl)methoxy]ethyl n-hexanoate.

5. A process as claimed in claim 1 (c) in which 3-[(2-azido-9H-purin-9-yl)methoxy]propyl acetate is converted to form 2-amino-9-[(3-hydroxypropoxy)methyl]-9H-purine.

6. A process as claimed in claim 1 (c) in which 2-amino-1,9-dihydro-9-[(2-benzyloxyethoxy)-methyl]-6H-purine-6-thione is converted into 2-amino-9-[(2-benzyloxyethoxy)methyl]9H-purine